(19) Europäisches Patentamt
European Patent Office
Office européen des brevets

(11) Numéro de publication: **0 103 503**
**A2**

(12)
# DEMANDE DE BREVET EUROPEEN

(21) Numéro de dépôt: **83401637.0**

(22) Date de dépôt: **10.08.83**

(51) Int. Cl.[3]: **A 61 K 31/395**, C 07 D 401/12, C 07 D 213/64, C 07 D 213/89, C 07 D 211/14, A 61 K 31/165, A 61 K 31/18, C 07 C 103/78, C 07 C 143/75, C 07 D 295/08 // C07D213/81, C07D211/46

(30) Priorité: **13.08.82 FR 8214128**

(43) Date de publication de la demande: **21.03.84 Bulletin 84/12**

(84) Etats contractants désignés: **AT BE CH DE FR GB IT LI LU NL SE**

(71) Demandeur: **SANOFI, société anonyme, 40, Avenue George V, F-75008 Paris (FR)**

(72) Inventeur: **Nisato, Dino, Viale Golgi, 80, Pavia (IT)**
Inventeur: **Boveri, Sergio, Corso Republica 48, Tortona (IT)**

(74) Mandataire: **Combe, André et al, CABINET BEAU DE LOMENIE 55 rue d'Amsterdam, F-75008 Paris (FR)**

(54) **Amides N-substitués, leurs sels, procédé pour leur préparation et compositions pharmaceutiques en contenant.**

(57) Amides N-substitués à action bloquant les récepteurs $H_2$ de l'histamine, de formule:

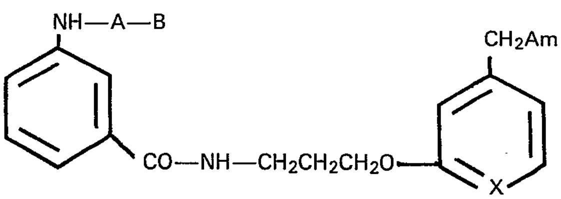

dans laquelle X est un atome d'azote ou un groupe CH; Am représente un groupe amino mono- ou disubstitué, un groupe pyrrolidino, morpholino, thiomorpholino, hexaméthylèneimino ou un groupe pipéridino ou pipérazino éventuellement substitués; A est CO ou $SO_2$ et B est un groupe alkyle contenant 1 à 6 atomes de carbone, un groupe phényle, un groupe pyridyle ou un groupe pyridyle 1-oxyde; leurs sels pharmaceutiquement acceptables; un procédé pour leur préparation; et compositions pharmaceutiques les renfermant, utiles dans le traitement de la maladie ulcéreuse.

EP 0 103 503 A2

Amides N-substitués, leurs sels, procédés pour leur préparation et compositions pharmaceutiques en contenant.

La présente invention concerne de nouveaux amides N-substitués ayant une activité bloquant les récepteurs $H_2$ de l'histamine, leurs sels, un procédé pour leur préparation, ainsi que des compositions pharmaceutiques renfermant lesdits composés en tant qu'ingrédients actifs.

Plus particulièrement, la présente invention c oncerne, selon un de ses aspects, des amides N-substitués de formule :

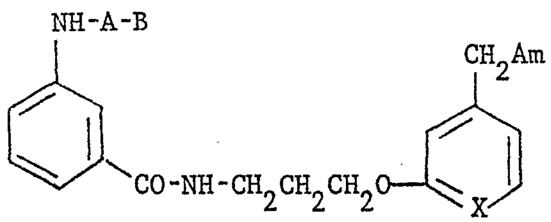

(I)

dans laquelle X représente un atome d'azote ou un groupe CH; Am représente un groupe amino substitué par un alkyle inférieur, par un alkyle inférieur portant un groupe hydroxy, phényle ou cycloalkyle ayant 3 à 6 atomes de carbone, ou par un radical choisi parmi alkényle inférieur, alkynyle inférieur, phényle et cycloalkyle ayant de 3 à 6 atomes de carbone; un groupe amino substitué par deux groupes alkényle inférieur ou alkynyle inférieur, identiques ou différents; un groupe amino substitué par deux groupes (hydroxy)alkyle inférieur ou (alkoxy inférieur)alkyle inférieur, identiques ou différents; un groupe dialkylamino inférieur; un groupe dialkylamino inférieur substitué par un groupe phényle, cycloalkyle ayant 3 à 6 atomes de carbone, méthoxyle, ou hydroxyle; un groupe pyrrolidino; un groupe morpholino; un groupe thiomorpholino; un groupe hexaméthylèneimino; un groupe pipéridino; un groupe pipéridino substitué par un groupe méthyle, hydroxyle ou hydroxyméthyle ou par deux groupes méthyle; un groupe pipérazino substitué en position 4 par un groupe alkyle inférieur ou (hydroxy)alkyle inférieur; ou un groupe pipérazino substitué en position 4 par un groupe alkyle inférieur ou (hydroxy)-alkyle inférieur et portant aussi un groupe méthyle dans les positions 3 et/ou 5; A est CO ou $SO_2$ et B est un groupe alkyle contenant 1 à 6 atomes de carbone, un groupe phényle, un groupe pyridyle

ou un groupe pyridyle 1-oxyde, ainsi que leurs sels pharmaceutiquement acceptables.

Le terme "dialkylamino inférieur", tel qu'utilisé ici, désigne le groupe $NH_2$ substitué par deux groupes alkyle inférieur, identiques ou différents.

Les termes "alkyle inférieur", "alkényle inférieur" et "alkynyle inférieur", tels qu'utilisés ici, désignent les radicaux hydrocarbonés aliphatiques, saturés ou contenant une double ou une triple liaison, ayant jusqu'à 4 atomes de carbone.

Le terme "alkoxy inférieur" désigne la fonction hydroxyle dont l'atome d'hydrogène est remplacé par un groupe alkyle inférieur, tel que défini ci-dessus.

Les termes "pyrrolidino", "pipéridino" et "pipérazino", tels qu'utilisés ici dans la description comme dans les revendications, remplacent la nomenclature recommandée par IUPAC pour les radicaux "1-pyrrolidinyl", "1-pipéridinyl" et, respectivement, "1-pipérazinyl". Le terme "hexaméthylèneimino" est utilisé ici pour désigner le radical "1H-hexahydroazépin-1-yl".

Après la subdivision des récepteurs de l'histamine en récepteurs $H_1$ (Ash et Schild, Br. J. Pharmacol. Chemother. 1966, 27, 427) et récepteurs $H_2$ (Black et al., Nature 1972, 236, 385) et la découverte que le blocage sélectif des récepteurs $H_2$ provoque une inhibition de la sécrétion gastrique, de nombreux produits ont été proposés comme antagonistes des récepteurs $H_2$ de l'histamine, ci-après indiqués "$H_2$-bloquants". Ainsi, les composés ayant reçu les Dénominations Communes Internationales burimamide, métiamide, cimétidine, ranitidine, tiotidine, étintidine, oxmétidine ont fait l'objet d'un grand nombre de publications scientifiques.

Tous les produits ci-dessus sont caractérisés par la présence dans leur molécule de la structure suivante :

$$-NH-\underset{\underset{Z}{\|}}{C}-NH- \qquad (II)$$

où Z représente un atome d'oxygène ou de soufre, ou bien un groupe N-CN, N-CO- ou $CH-NO_2$, ladite structure étant linéaire et liée à deux groupements aliphatiques ou incluse dans un cycle comme dans le cas de l'oxmétidine.

La cimétidine, 2-cyano-1-méthyl-3-[2-[[(5-méthyl-imidazol-4-yl)méthyl]thio]éthyl]guanidine, comportant la structure II où Z est N-CN, et la ranitidine, N-[2-[[5-[(diméthylamino)-méthyl]furfuryl]thio]éthyl]-N'-méthyl-2-nitro-1,1-éthènediamine, comportant la structure II où Z est $CH-NO_2$, sont déjà utilisées en thérapeutique pour le traitement de l'ucère gastrique et duodénal.

La demande de brevet européen publiée 23 578 décrit des dérivés de l'aminoalkylbenzène de formule :

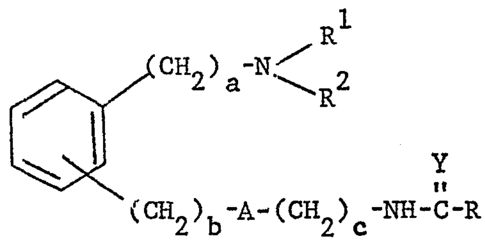

(III)

utiles comme $H_2$-bloquants.

Plus particulièrement, ladite demande de brevet indique que les composés de formule III, disubstitués en 1,3 sur le noyau benzène, où $NR^1R^2$ est pyrrolidino, a = 1, b = 0, A = oxygène, c = 3, Y = oxygène et R = hydrogène ou 3-pyridyle (composé A et composé B ci-dessous) possèdent une DE50, respectivement de 0,84 et 1,25 mg/kg/i.v. dans le test de l'hypersécrétion gastrique, selon Ghosh et Schild.

Ladite demande de brevet comprend d'une façon générale les composés de formule III, dans laquelle R est un groupe aryle substitué par un groupe alcanesulfonamido contenant 1 à 5 atomes de carbone ou alcanoylamino contenant 1 à 5 atomes de carbone et, parmi les nombreux exemples, elle cite les composés de formule

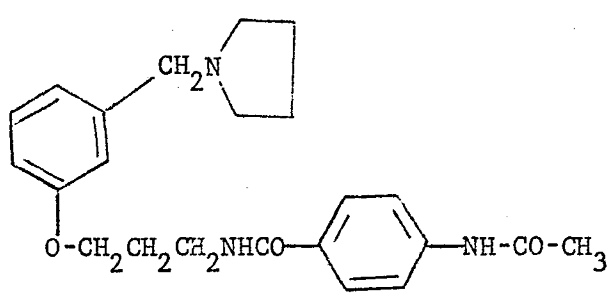

(IV)

et de formule

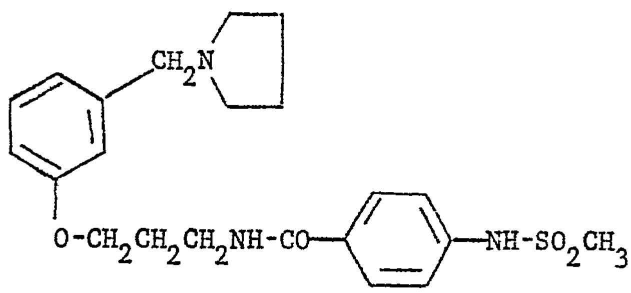

(V)

On a maintenant trouvé que les composés de formule I ci-dessus, caractérisés par la présence d'un amide de l'acide benzoïque substitué en position méta par un groupe acylamido ou sulfonamido, possèdent une activité $H_2$-bloquante extraordinairement élevée.

On a aussi trouvé d'une façon surprenante que la substitution en position méta est essentielle pour l'obtention d'une activité élevée, comme il a été démontré par un essai de comparaison entre, entre autres, les composés de l'invention de formule I, où Am est pyrrolidino, A est CO ou $SO_2$ et B est $CH_3$ et leurs isomères para de formules IV et V ci-dessus, décrits dans la demande de brevet européen publiée 23 578.

La sélectivité de l'activité des produits de la présente invention vers les récepteurs du type $H_2$ est confirmée par l'absence d'activité du type $H_1$ dans le test de la contraction provoquée par l'histamine sur l'iléon isolé de cobaye.

L'activité antagoniste des composés de la présente invention vers les récepteurs $H_2$ gastriques de l'histamine a été confirmée dans le test de l'activité antisécrétoire basé sur l'antagonisme pour l'hypersécrétion provoquée par l'histamine chez le rat atropinisé, selon la méthode de Ghosh et Schild (Br..J. Pharmacol. Chemother. 1958, 13, 54) modifiée selon Black (Nature 1972, 236, 385). Selon ce test, on provoque un hypersécrétion acide gastrique par infusion intraveineuse d'une dose submaximale d'histamine équivalant à 15 mcmol/kg/h et l'on mesure la sécrétion gastrique par perfusion d'une solution physiologique à une vitesse constante dans l'estomac de l'animal.

Comme composés de référence, on a utilisé la cimétidine et la ranitidine, qui représentent les composés standards, ainsi que :

- le composé de formule :

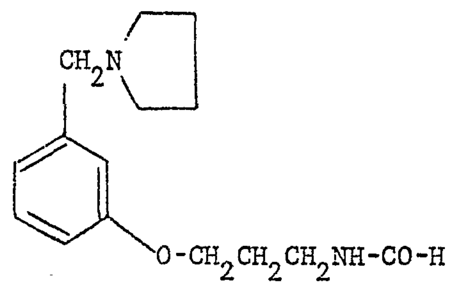

sous forme d'oxalate, ci-après désigné "Composé A";
- le composé de formule :

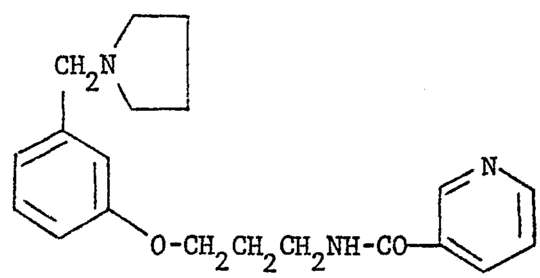

sous forme de dioxalate, ci-après désigné "Composé B";
décrits dans la demande de brevet européen publiée 23 578 comme produits particulièrement actifs; et
- Les composés IV (sous forme de base libre) et V (sous forme d'oxalate) ci-dessus, également décrits dans la demande de brevet européen publiée 23 578.

Comme composés représentatifs de la présente invention, on a utilisé :
- le composé de formule

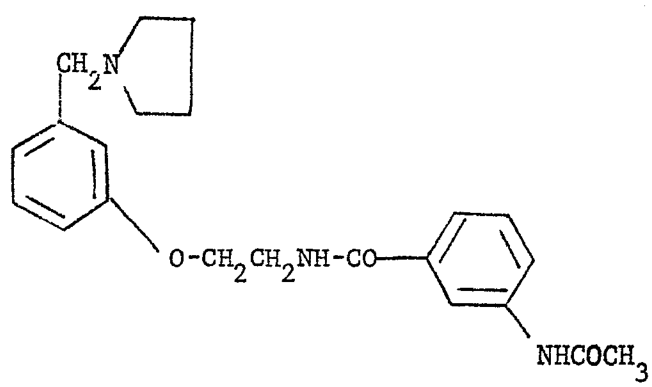

sous forme de base libre, désigné par son numéro de code SR 58096 (exemple 13 ci-dessous), isomère méta du composé IV;

- le composé de formule

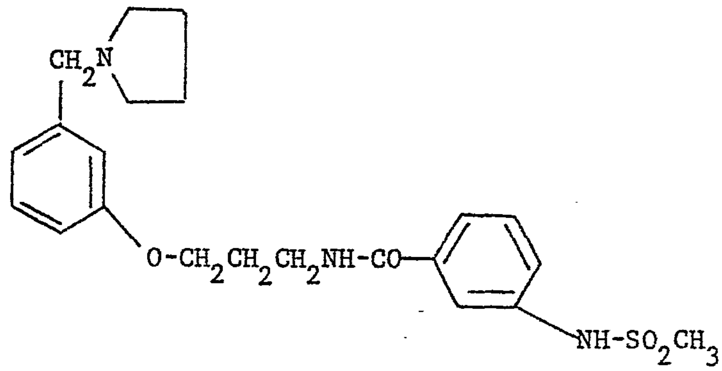

sous forme d'oxalate, désigné par son numéro de code SR 57896A (exemple 1 ci-dessous), isomère méta du composé V, ainsi que les composés désignés par leur numéro de code SR 57919, SR 57958, SR 58032, SR 58070 et SR 58079A, décrits respectivement, dans les exemples 2, 4, 10, 20 et 21 ci-dessous.

Le tableau I montre, pour chaque produit, la dose (en mcmol/kg par voie veineuse en dose unique) qui inhibe de 50% l'hypersécrétion gastrique provoquée par l'histamine (DI50) ainsi que la puissance relative par rapport à la cimétidine.

TABLEAU I

| Composé | DI50 (mcmol/kg) | Puissance relative (cimétidine = 1) |
|---|---|---|
| Cimétidine | 0,95 | 1,00 |
| Ranitidine | 0,25 | 3,80 |
| Composé A | 2,01 | 0,47 |
| Composé B | 2,22 | 0,43 |
| Composé IV | 5,36 | 0,18 |
| SR 58096 | 1,12 | 0,85 |
| Composé V | 9,35 | 0,10 |
| SR 57896A | 0,52 | 1,82 |
| SR 57919 | 0,16 | 5,93 |
| SR 57958 | 0,27 | 3,51 |
| SR 58032 | 0,08 | 11,87 |
| SR 58070 | 0,56 | 1,70 |
| SR 58079A | 0,025 | 38,00 |

De ce tableau, il ressort que les composés représentatifs de la présente invention sont très actifs et qu'un d'entre

eux est même 10 fois plus actif que la ranitidine. En outre, ils sont beaucoup plus actifs que les quatre produits décrits dans la demande de brevet européen publiée 23 578. Plus particulièrement, le SR 58096 est environ 5 fois plus actif que le composé IV et le SR 57896A est environ 18 fois plus actif que le composé V. Ces dernières données sont très surprenantes si l'on considère que la différence entre les composés de l'invention et les composés IV et V n'est qu'une isomérie de position.

Les composés de formule I ci-dessus ainsi que leurs sels pharmaceutiquement acceptables sont préparés, selon un autre aspect de la présente invention, suivant un procédé caractérisé en ce qu'on traite une amine de formule

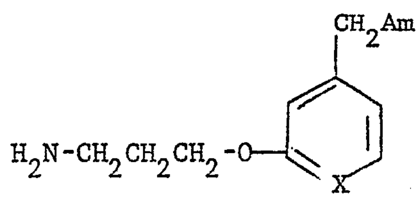

(VI)

dans laquelle X et Am sont tels que définis ci-dessus, avec un dérivé fonctionnel d'un acide carboxylique de formule

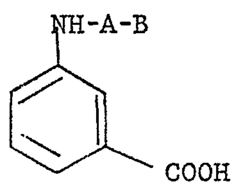

(VII)

dans laquelle A et B sont tels que définis ci-dessus, dans un solvant organique à une température comprise entre 0°C et la température d'ébullition du solvant employé et l'on transforme éventuellement le produit ainsi obtenu dans ses sels pharmaceutiquement acceptables.

Comme dérivé fonctionnel approprié, on peut utiliser l'acide libre activé, l'anhydride, un anhydride mixte, le chlorure ou un ester actif.

Un dérivé fonctionnel convenable de l'acide de formule VII ci-dessus est un ester actif de formule :

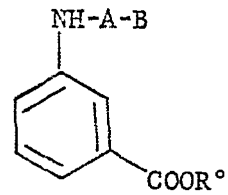

(VIII)

dans laquelle R° représente un groupe nitrophényle, méthoxyphényle, trityle, benzhydryle.

La température de réaction peut varier entre 0°C et la température d'ébullition du solvant employé, mais en général on opère à la température ambiante ou à 30-50°C. Il peut être préférable de conduire la réaction au froid lorsqu'elle est exothermique, par exemple lorsqu'on utilise le chlorure en tant que dérivé fonctionnel de l'acide carboxylique de formule VII.

Comme solvant de réaction, on utilise de préférence un alcool, tel que le méthanol, ou un solvant halogéné tel que le chlorure de méthylène, le dichloroéthane, le chloroforme et similaires, mais d'autres solvants organiques compatibles avec les réactifs employés, par exemple l'acétonitrile, le dioxane, le tétrahydrofuranne ou un hydrocarbure tel que l'hexane peuvent être également employés.

La réaction peut être conduite en présence d'un accepteur de protons, par exemple d'un carbonate alcalin ou d'une amine tertiaire, lorsque de l'acide chlorhydrique ou un autre acide se libère pendant la réaction, mais cet accepteur de protons n'est pas indispensable pour l'obtention du produit final.

La réaction est assez rapide; en général, après 1 à 4 h à la température ambiante ou à 30-50°C, la réaction est achevée et le composé de formule I obtenu est isolé selon les techniques conventionnelles sous forme de base libre ou d'un de ses sels.

La base libre peut être transformée dans un de ses sels pharmaceutiquement acceptables par traitement avec une solution de l'acide convenable dans un solvant organique. Si le composé I est isolé sous forme de sel, la base libre correspondante peut être libérée à l'aide d'un hydroxyde ou d'un carbonate alcalin.

Les composés de départ de formule VI ci-dessus, où X est CH, sont connus en littérature ou bien ils peuvent être aisément préparés à partir du 3-hydroxybenzaldéhyde (IX) par amination réductive avec l'amine Am-H (X) en utilisant le borohydrure de sodium dans du méthanol comme agent de réduction et par réaction de l'aminophénol (XI) ainsi obtenu avec le chlorhydrate de 3-chloropropylamine en présence d'hydrure de sodium dans le diméthylformamide, selon le schéma suivant :

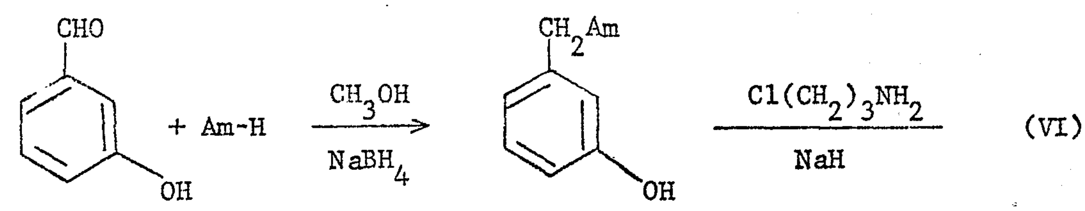


(IX) (X) (XI)

dans lequel Am est tel que défini ci-dessus.

Les composés de départ de formule VI ci-dessus, où X est N, sont connus ou bien ils peuvent être préparés à partir de l'acide 2-chloroisonicotinique (XII) en le transformant d'abord dans le chlorure d'acide par action du chlorure de thionyle et ensuite dans l'amide substitué (XIII) par réaction avec l'amine Am-H (X), en faisant réagir l'amide (XIII) ainsi obtenu avec le 3-hydroxypropyl-2,5-diméthylpyrrole, en soumettant l'amine bloquée (XIV) ainsi obtenue à une réduction avec l'hydrure de lithium et d'aluminium et en faisant réagir l'aminométhylpyridyloxypropylpyrrole (XV) ainsi obtenu avec le chlorhydrate d'hydroxylamine pour libérer l'amine VI, selon le schéma suivant :

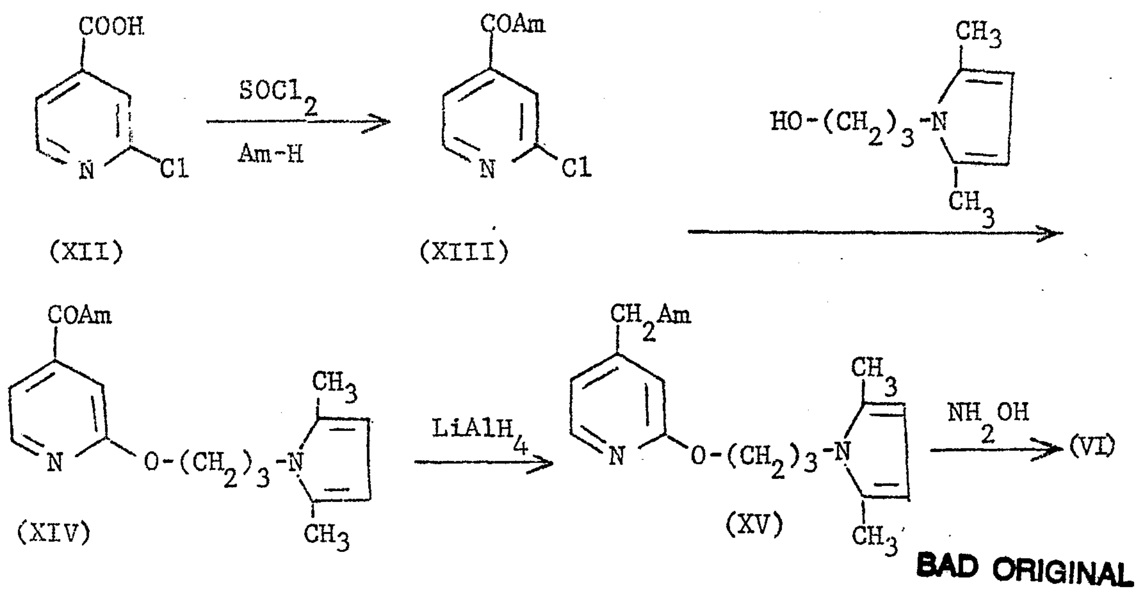


(XII) (XIII) (XIV) (XV)

BAD ORIGINAL

Les composés de formule VIII ci-dessus sont connus en littérature ou bien ils peuvent être aisément préparés par réaction de l'acide VII dans l'alcool ou le phénol approprié en présence d'un agent de condensation tel que dicyclohexylcarbodiimide, dans un solvant tel que le chlorure de méthylène.

Les composés de la présente invention sont peu toxiques et sont utiles comme médicaments.

Ainsi, selon un autre de ses aspects, la présente invention se réfère à des compositions pharmaceutiques renfermant, en tant qu'ingrédients actifs, les composés de formule I ci-dessus, ainsi que leurs sels d'addition pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous des formes unitaires d'administration en mélange avec des supports pharmaceutiques classiques, aux animaux et aux êtres humains pour le traitement de l'hypersécrétion gastrique et de la maladie ulcéreuse. Les formes unitaires d'administration appropriées comprennent les formes par voie orale telles que les comprimés, les gélules, les poudres, les granules et les solutions ou suspensions orales et les formes d'administration sublinguale et buccale, les formes d'administration parentérale utiles pour une injection sous-cutanée, intramusculaire ou intraveineuse, ainsi que les formes d'administration rectale.

Afin d'obtenir l'effet antisécrétoire désiré, la dose de principe actif peut varier entre 0,1 et 100 mg/kg de poids du corps et par jour.

Chaque dose unitaire peut contenir de 10 à 1 000 mg, de 50 à 500 mg de préférence, d'ingrédient actif en combinaison avec un support pharmaceutique. Cette dose unitaire peut être administrée 1 à 4 fois par jour pour traiter l'hypersécrétion gastrique et la maladie ulcéreuse.

Les exemples suivants illustrent l'invention sans toutefois la limiter.

PREPARATION 1

a) 3-morpholinométhylphénol.

A une solution de 0,2 mol de 3-hydroxybenzaldéhyde dans 100 ml de méthanol, on ajoute, à environ 20°C, une solution de 0,4 mol de morpholine dans 40 ml de méthanol. Dans le mélange obtenu, on introduit par portions, sous agitation, à la température de 20-25°C et en 90 min environ, 0,2 mol de borohydrure de sodium, puis on agite le mélange réactionnel 3 h à la température ambiante et on évapore le solvant sous pression réduite jusqu'à siccité. On reprend le résidu par de la glace, on l'acidifie avec de l'acide chlorhydrique, on lave la solution acide deux fois avec 40 ml d'acétate d'éthyle et on y ajoute de l'hydroxyde d'ammonium concentré jusqu'à réaction nettement basique. On extrait quatre fois avec 80 ml d'acétate d'éthyle et on évapore le solvant. Le produit ainsi obtenu fond à 113-116°C. Rendement : 67%.

b) 3-(3-morpholinométhylphénoxy)propylamine.

A une suspension de 0,28 mol d'hydrure de sodium à 80% dans du diméthylformamide, on ajoute par portions, sous agitation, à la température d'environ 20°C, 0,06 mol de 3-morpholinométhylphénol, puis on agite 15 min à la température ambiante et on ajoute ensuite au mélange, à 5-10°C, une solution de 0,08 mol de chlorhydrate de 3-chloropropylamine dans 20 ml de diméthylformamide. On agite le mélange réactionnel 24 h à la température ambiante, puis on y ajoute 400 ml d'eau glacée et on extrait quatre fois avec 100 ml d'acétate d'éthyle. On lave la solution organique deux fois avec 30 ml d'hydroxyde de sodium à 10% et ensuite trois fois avec 20 ml d'eau. On sèche sur du sulfate de sodium anhydre et on évapore à sec sous pression réduite. On obtient ainsi le 3-(3-morpholinométhylphénoxy)propylamine sous forme d'une huile jaune. Rendement : 64%.

PREPARATION 2

3-(3-thiomorpholinométhylphénoxy)propylamine.

En opérant comme décrit ci-dessus par réaction de 0,2 mol de 3-hydroxybenzaldéhyde avec 0,4 mol de thiomorpholine en présence de 0,2 mol de borohydrure de sodium (comme dans la préparation 1a), on obtient le 3-thiomorpholinométhylphénol qui,

BAD ORIGINAL

traité avec un excès de chlorhydrate de 3-chloropropylamine en présence d'hydrure de sodium (comme dans la préparation 1b), donne le 3-(3-thiomorpholinométhylphénoxy)propylamine, sous forme d'huile.

PREPARATION 3

a) 3-(4-hydroxypipéridinométhyl)phénol.

A une solution de 0,2 mol de 3-hydroxybenzaldéhyde dans 100 ml de méthanol, on ajoute à environ 20°C une solution de 0,4 mol de 4-hydroxypipéridine dans 80 ml de méthanol. Dans le mélange obtenu, on introduit, par portions et sous agitation, à la température de 20-25°C et en 90 min environ, 0,2 mol de borohydrure de sodium, puis on agite le mélange réactionnel 4 h à la température ambiante et on évapore le solvant sous pression réduite jusqu'à siccité. On reprend le résidu par de la glace, on acidifie avec de l'acide chlorhydrique, on lave la solution acide deux fois avec 40 ml d'acétate d'éthyle et on y ajoute de l'hydroxyde d'ammonium concentré jusqu'à réaction nettement basique. On filtre le produit précipité et on le cristallise dans 400 ml d'eau. Le produit ainsi obtenu fond à 156-158°C. Rendement : 66,8%.

b) 3-[3-(4-hydroxypipéridinométhyl)phénoxy]propylamine.

A une suspension de 0,36 mol d'hydrure de sodium à 50% dans 180 ml de diméthylformamide, on ajoute par portions sous agitation, à la température d'environ 20°C, 0,14 mol de 3-(4-hydroxypipéridinométhyl)phénol, puis on agite 15 min à 40°C et, ensuite, on ajoute au mélange, à 5-10°C, une solution de 0,18 mol de chlorhydrate de 3-chloropropylamine dans 80 ml de diméthylformamide. On agite le mélange réactionnel 24 h à la température ambiante, puis on y ajoute 400 ml d'eau glacée contenant de l'acide chlorhydrique et on ajuste le pH de la solution à 6. On lave la solution obtenue deux fois avec 100 ml d'acétate d'éthyle et on l'alcalinise avec de l'hydroxyde de sodium concentré. On extrait quatre fois avec 80 ml d'acétate d'éthyle, on sèche la phase organique sur du sulfate de sodium anhydre et on évapore à sec sous pression réduite. On chromatographie l'huile obtenue avec du méthanol et, par évaporation de la fraction pure, on isole le produit sous forme d'une huile jaune. Rendement : 16%.

PREPARATIONS 4 à 13

En opérant comme décrit ci-dessus, par réaction de 0,2 mol de 3-hydroxybenzaldéhyde avec 0,4 mol de, respectivement, pipéridine, 4-méthylpipéridine, 3-méthylpipéridine, hexaméthylène-imine, méthylbenzylamine, 3-hydroxyméthylpipéridine, 4-méthyl-pipérazine, diméthylamine, di-n-propylamine, di-n-butylamine en présence de 0,2 mol de borohydrure de sodium (comme dans la préparation 3a), on obtient les aminométhylphénols correspondants qui, traités avec un excès de chlorhydrate de 3-chloropropylamine en présence d'hydrure de sodium (comme dans la préparation 3b), donnent les composés correspondants de formule VI ci-dessus.

Les caractéristiques des aminométhylphénols XI et des intermédiaires VI ainsi obtenus sont résumées dans le tableau II.

TABLEAU II

| Prépa-ration | Am | Caractéristiques physicochimiques | |
|---|---|---|---|
| | | Composé XI | Composé VI |
| 4 | 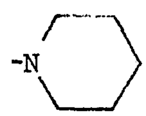 | F. 128-130°C | F. 202-205°C (dichlorhydrate) |
| 5 | 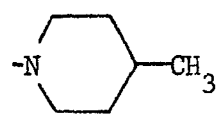 | huile jaune pâle | F. 148-151°C (dioxalate) |
| 6 | 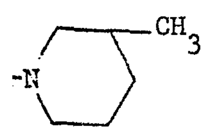 | F. 155-157°C | huile jaune |
| 7 | 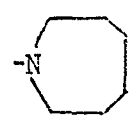 | F. 116-118°C | huile jaune |
| 8 | 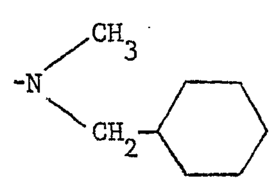 | F. 206-211°C (chlorhydrate) | F. 147-149°C (chlorhydrate) |
| 9 | 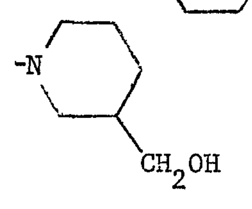 | huile | huile |

TABLEAU II (suite)

| Préparation | Am | Caractéristiques physicochimiques | |
|---|---|---|---|
| | | Composé XI | Composé VI |
| 10 | 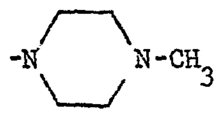 | F. 255-258°C (chlorhydrate) | F. 253-255°C (trichlorhydrate) |
| 11 | 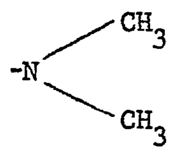 | F. 106-109°C | F. 213-216°C (dichlorhydrate) |
| 12 | 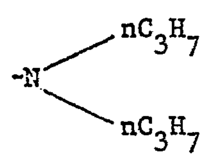 | F. 184-186°C (chlorhydrate) | F. 204-206°C (dichlorhydrate) |
| 13 | 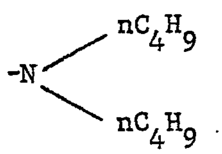 | F. 50-55°C | huile |

PREPARATION 14

a) 2-chloro-4-pipéridinocarbonylpyridine.

On chauffe au reflux pendant 2 h un mélange de 0,13 mol d'acide 2-chloroisonicotinique et 70 ml de chlorure de thionyle, puis on concentre sous pression réduite et on reprend le résidu avec du toluène anhydre. On évapore le solvant et l'on suspend le chlorure de l'acide 2-chloroisonicotinique brut ainsi obtenu sous forme d'un solide jaune, dans 50 ml de chlorure de méthylène. On refroidit le mélange à 0-5°C et on y ajoute lentement une solution de 0,3 mol de pipéridine dans 50 ml de chlorure de méthylène. On agite 2 h à la température ambiante, on lave à fond à l'eau et l'on évapore à sec la phase organique. On obtient ainsi 19 g de 2-chloro-4-pipéridinocarbonylpyridine qui est purifiée par chromatographie sur gel de silice en éluant avec de l'acétate d'éthyle. On obtient ainsi 10 g de produit pur sous forme d'une huile jaune.

b) 2-[3-(2,5-diméthylpyrrol-1-yl)propoxy]-4-pipéridino-carbonyl-pyridine.

A une suspension de 0,047 mol de 2,5 diméthyl-1-(3-hydroxypropyl)pyrrole et 0,059 mol d'hydrure de sodium à 80% dans 150 ml de 1,2-diméthoxyéthane, chauffée 15 min au reflux au préalable et refroidie, on ajoute 0,044 mol de 2-chloro-4-pipéridinocarbonyl-pyridine, on chauffe ensuite le mélange réactionnel 2 h au reflux et on l'évapore à sec. On reprend le résidu avec de l'eau, on extrait avec de l'acétate d'éthyle, on sépare le phase organique et l'on évapore à sec. On obtient ainsi 15,4 g de produit huileux.

c) 2-[3-(2,5-diméthylpyrrol-1-yl)propoxy]-4-pipéridino-méthylpyridine.

On chauffe au reflux pendant 1 h un mélange de 15,4 g du produit ainsi obtenu, 2 g d'hydrure de lithium et d'aluminium et 150 ml de 1,2-diméthoxyéthane, puis on refroidit et on ajoute lentement de l'eau au mélange. On filtre, on évapore à sec la solution obtenue, on reprend le résidu avec de l'eau et on acidifie avec de l'acide chlorhydrique N. On lave la solution deux fois avec 30 ml d'acétate d'éthyle, on ajoute de l'hydroxyde de sodium jusqu'à réaction basique et l'on extrait quatre fois avec 30 ml d'acétate d'éthyle. On sèche la phase organique sur du sulfate de sodium anhydre et l'on évapore à sec. On obtient ainsi 8,7 g de produit huileux.

d) 3-(4-pipéridinométhylpyrid-2-yloxy)propylamine.

A une solution de 8,6 g de chlorhydrate d'hydroxylamine dans 26 ml d'éthanol, sous agitation à la température ambiante depuis 30 min, on ajoute une solution de 2,8 g d'hydroxyde de potassium à 85% dans 10 ml d'éthanol à 50%, puis on agite encore 10 min et on ajoute 8,7 g de 2-[3-(2,5-diméthylpyrrol-1-yl)propoxy]-4-pipéridinométhylpyridine. On chauffe au reflux pendant 6 h, puis on filtre, on lave avec de l'éthanol, on acidifie par de l'acide chlorhydrique N et l'on lave deux fois avec 30 ml d'acétate d'éthyle. On ajuste le pH de la solution aqueuse à 8, on lave encore deux fois avec 30 ml d'acétate d'éthyle et l'on rend la solution nettement basique par addition d'une solution concentrée d'hydroxyde de potassium. On extrait avec de l'acétate d'éthyle, on sèche la phase organique sur du sulfate de sodium anhydre et on évapore. On obtient ainsi

5,3 g de 3-(4-pipéridinométhylpyrid-2-yloxy)propylamine sous forme d'huile.

PREPARATION 15

On traite l'acide 2-chloroisonicotinique avec le chlorure de thionyle et, ensuite, le chlorure d'acide ainsi obtenu avec la diméthylamine (comme décrit dans la préparation 14a), pour obtenir le diméthylamide de l'acide 2-chloroisonicotinique qui, par condensation avec 2,5-diméthyl-1-(3-hydroxypropyl)pyrrole en présence d'hydrure de sodium (comme décrit dans la préparation 14b), donne le N,N-diméthyl-2-[3-(2,5-diméthylpyrrol-1-yl)propoxy]isonicotinamide (huile). On réduit le produit ainsi obtenu avec l'hydrure de lithium et d'aluminium (comme décrit dans la préparation 14c) et l'on débloque le 2-[3-(2,5-diméthylpyrrol-1-yl)-propoxy]-4-diméthylaminométhylpyridine par action de l'hydroxylamine (comme décrit dans la préparation 14d) pour isoler le 3-(4-diméthylaminométhylpyrid-2-yloxy)-propylamine sous forme d'une huile.

PREPARATIONS 16 à 33

En opérant comme décrit dans la préparation 1, par réaction de 0,2 mol de 3-hydroxybenzaldéhyde avec 0,2 mol de l'amine appropriée en présence de 0,2 mol de borohydrure de sodium et par traitement du produit ainsi obtenu avec le chlorhydrate de 3-chloropropylamine en présence d'hydrure de sodium, on obtient :

- la 3-(3-butylaminométhylphénoxy)propylamine (16),
- la 3-(3-allylaminométhylphénoxy)propylamine (17),
- la 3-(3-propargylaminométhylphénoxy)propylamine (18),
- la 3-[(2-hydroxyéthyl)aminométhylphénoxy]propylamine (19),
- la 3-(3-benzylaminométhylphénoxy)propylamine (20),
- la 3-(3-cyclopropylméthylaminométhylphénoxy)propylamine (21),
- la 3-(3-phénylaminométhylphénoxy)propylamine (22),
- la 3-(3-cyclopentylaminométhylphénoxy)propylamine (23),
- la 3-(3-diallylaminométhylphénoxy)propylamine (24),
- la 3-(3-dipropargylaminométhylphénoxy)propylamine (25),
- la 3-[3 -di(2-hydroxyéthyl)aminométhylphénoxy]propylamine (26),
- la 3-[3-di(2-méthoxyéthyl)aminométhylphénoxy]propylamine (27),
- la 3-[(méthylcyclopropylméthyl)aminométhylphénoxy]propylamine (28),

- la 3-[4-(2-hydroxyéthylpipérazino)méthylphénoxy]propylamine (29),
- la 3-(3,4,5-triméthylpipérazinométhylphénoxy)propylamine (30),
- la 3-(3,4-diméthylpipérazinométhylphénoxy)propylamine (31),
- la 3-(3,5-diméthylpipéridinométhylphénoxy)propylamine (32) et, respectivement,
- la 3-[4-(2-hydroxyéthyl)-3-méthylpipérazinophénoxy]propylamine (33).

EXEMPLE 1

A une solution de 0,03 mol de 3-(3-pyrrolidinométhylphénoxy)propylamine dans 200 ml de méthanol, on ajoute 0,03 mol de 3-méthanesulfonamidobenzoate de p-nitrophényle et on agite le mélange obtenu 2 h à 60-70°C. Après évaporation du solvant, on reprend le résidu par 60 ml d'acide chlorhydrique N. On lave la solution trois fois avec 20 ml d'acétate d'éthyle, puis on ajuste le pH de la solution aqueuse à 8,1 par de l'hydroxyde de sodium dilué et l'on extrait quatre fois avec 30 ml d'acétate d'éthyle. On évapore le solvant, on dissout le résidu huileux dans de l'éthanol et l'on traite la solution ainsi obtenue avec une solution d'acide oxalique en éthanol. Par cristallisation du précipité dans l'éthanol à 95%, on obtient 4 g d'oxalate de 3-méthanesulfonamido-N-[3-(3-pyrrolidinométhylphénoxy)propyl]benzamide, SR 57896A; F. 180-181°C.

EXEMPLE 2

En opérant comme décrit dans l'exemple 1, par réaction de 0,03 mol de 3-(3-pyrrolidinométhylphénoxy)propylamine avec 0,03 mol de 3-benzènesulfonamidobenzoate de p-nitrophényle dans 300 ml de méthanol, on obtient le 3-benzènesulfonamido-N-[3-(3-pyrrolidinométhylphénoxy)propyl]benzamide, SR 57919, isolé sous forme de base libre. Solide vitreux.

EXEMPLE 3

A une solution de 0,03 mol de 3-(3-diméthylaminométhylphénoxy)propylamine dans 200 ml de méthanol, on ajoute 0,03 mol de 3-méthanesulfonamidobenzoate de p-nitrophényle, puis on agite le mélange obtenu 2 h à 60-70°C. En opérant ensuite comme décrit dans l'exemple 1, on obtient l'oxalate de 3-méthanesulfonamido-N-[3-(3-diméthylaminométhylphénoxy)propyl]benzamide, SR 57909A; F. 146-149°C.

De la même façon, à partir de la 3-(4-diméthylaminométhylpyrid-2-yloxy)propylamine, on obtient l'oxalate de 3-méthane-

BAD ORIGINAL

sulfonamido-N-[3-(4-diméthylaminométhylpyrid-2-yloxy)propyl]-benzamide.

EXEMPLE 4

A une solution de 0,03 mol de 3-(3-diméthylaminométhylphénoxy)propylamine dans 300 ml de méthanol, on ajoute 0,03 mol de 3-(3-pyridine 1-oxyde)carboxamidobenzoate de p-nitrophényle et on agite le mélange obtenu 2 h à 60-70°C. Après évaporation du solvant, on reprend le résidu par 60 ml d'acide chlorhydrique N et l'on lave la solution trois fois avec 20 ml d'acétate d'éthyle, puis on ajuste le pH de la solution acide à 8,2 et l'on extrait quatre fois avec 30 ml d'acétate d'éthyle. On évapore à sec et l'on lave le résidu avec de l'éther diéthylique. On obtient ainsi le 3-[(3-pyridine 1-oxyde)carboxamido]-N-[3-(3-diméthylaminométhylphénoxy)propyl]-benzamide, SR 57958; F. 170-173°C.

EXEMPLES 5 à 9

En opérant comme décrit dans l'exemple 1, par réaction de 0,03 mol de 3-(3-thiomorpholinométhylphénoxy)propylamine, de 3-(3-morpholinométhylphénoxy)propylamine, de 3-(3-hexaméthylèneiminométhylphénoxy)propylamine, de 3-(3-di-n-propylaminométhylphénoxy)-propylamine et de 3-(3-di-n-butylaminométhylphénoxy)propylamine avec 0,03 mol de 3-méthanesulfonamidobenzoate de 4-nitrophényle dans du méthanol, on obtient, respectivement :

- l'oxalate de 3-méthanesulfonamido-N-[3-(3-thiomorpholinométhylphénoxy)propyl]benzamide (exemple 5);
- l'oxalate de 3-méthanesulfonamido-N-[3-(3-morpholinométhylphénoxy)-propyl]benzamide (exemple 6);
- l'oxalate de 3-méthanesulfonamido-N-[3-(3-hexaméthylèneiminométhylphénoxy)propyl]benzamide (exemple 7);
- l'oxalate de 3-méthanesulfonamido-N-[3-(3-di-n-propylaminométhylphénoxy)propyl]benzamide (exemple 8); et
- l'oxalate de 3-méthanesulfonamido-N-[3-(3-di-n-butylaminométhylphénoxy)propyl]benzamide (exemple 9).

EXEMPLE 10

A une solution de 0,023 mol de 3-(3-pipéridinométhylphénoxy)propylamine dans 150 ml d'éthanol, on ajoute, par portions, 0,023 mol de 3-méthanesulfonamidobenzoate de 4-nitrophényle. En opérant ensuite comme décrit dans l'exemple 1, on obtient le 3-méthane-

sulfonamido-N-[3-(3-pipéridinométhylphénoxy)propyl]benzamide, SR 58032; F. 88-90°C.

EXEMPLES 11 et 22

En opérant comme décrit dans l'exemple 10, par réaction de 0,03 mol de 3-[3-(4-méthylpipéridinométhyl)phénoxy]propylamine et de 3-[3-(4-hydroxypipéridinométhyl)phénoxy]propylamine avec 0,03 mol de 3-méthanesulfonamidobenzoate de 4-nitrophényle, on obtient, respectivement :

- le 3-méthanesulfonamido-N-[3-[3-(4-méthylpipéridinométhyl)phénoxy]-propyl]benzamide (exemple 11) et
- le 3-méthanesulfonamido-N-[3-[3-(4-hydroxypipéridinométhyl)phénoxy]-propyl]benzamide (exemple 12).

EXEMPLE 13

A une solution de 0,03 mol de 3-(3-pyrrolidinométhylphénoxy)propylamine dans 100 ml de méthanol, on ajoute, par portions, 0,03 mol d'acétamidobenzoate de 4-nitrophényle, puis on opère comme décrit dans l'exemple 1. On obtient ainsi le 3-acétamido-N-[3-(3-pyrrolidinométhylphénoxy)propyl]benzamide sous forme de base libre, SR 58096; F. 94-97°C.

EXEMPLES 14 à 16

En opérant comme décrit dans l'exemple 10, par réaction de 0,03 mol de 3-(3-benzylméthylaminométhylphénoxy)propylamine, de 3-[3-(3-hydroxyméthyl)pipéridinométhylphénoxy]propylamine et de 3-[3-(4-méthylpipérazinométhyl)phénoxy]propylamine avec 0,03 mol de 3-méthanesulfonamidobenzoate de 4-nitrophényle dans de l'éthanol, on obtient, respectivement :

- le 3-méthanesulfonamido-N-[3-(3-benzylméthylaminométhylphénoxy)-propyl]benzamide (exemple 14);
- le 3-méthanesulfonamido-N-[3-[3-(3-hydroxyméthyl)pipéridinométhylphénoxy]-propyl]benzamide (exemple 15); et
- le 3-méthanesulfonamido-N-[3-[3-(4-méthylpipérazinométhyl)phénoxy]-propyl]benzamide sous forme de chlorhydrate (exemple 16).

EXEMPLE 17

A une solution de 5 g de 4-diméthylaminopyridine en 80 ml d'acétonitrile, on ajoute 9,07 g d'acide 3-méthanesulfonamidobenzoïque. On agite le mélange 10 min à la température ambiante, puis on y ajoute 9,2 g de dicyclohexylcarbodiimide. Au bout de 5 min à la température ambiante, on ajoute 10 g de 3-(3-pipéridinométhylphénoxy)-propylamine dissoute dans 20 ml d'acétonitrile. On note une réaction

faiblement exothermique (de 20 à 25°C) et une dissolution qui, au bout de 5 min, est presque complète. On agite ensuite le mélange 4 h à 40°C, on filtre la dicyclohexylurée qui s'est formée et on lave avec de l'acétonitrile. On évapore à sec sous pression réduite et l'on purifie le résidu. On obtient ainsi le 3-méthanesulfonamido-N-[3-(3-pipéridinométhylphénoxy)propyl]benzamide, identique au produit de l'exemple 10.

EXEMPLE 18

A une suspension de 4,6 g d'acide 3-éthanesulfonamido-benzoïque, 2,5 g de diméthylaminopyridine, 8,8 g d'hexafluorophosphate de benzotriazolyloxy-tris-(diméthylamino)phosphonium dans 40 ml d'acétonitrile, on ajoute, à 20°C et sous agitation, 5,2 g de 3-[3-(3-méthylpipéridinométhyl)phénoxy]propylamine dissoute dans 10 ml d'acétonitrile. La température augmente jusqu'à 30°C. On agite la solution ainsi obtenue pendant 2 h à la température ambiante. On concentre le mélange sous pression réduite, on traite le résidu avec 20 ml d'hydroxyde de sodium concentré et on l'extrait quatre fois avec 40 ml d'acétate d'éthyle. Après l'évaporation des extraits organiques, on purifie le résidu. On obtient ainsi le 3-éthanesulfonamido-N-[3-[3-(3-méthylpipéridinométhyl)phénoxy]propyl]benzamide sous forme d'huile.

EXEMPLE 19

A un mélange de 0,03 mol de 3-(4-pipéridinométhyl-pyrid-2-yloxy)propylamine et 0,095 mol de pyridine dans 100 ml de chlorure de méthylène, agité à 0°C, on ajoute, par portions, 0,03 mol de 3-méthanesulfonamidobenzoate de 4-nitrophényle, puis on opère comme décrit dans l'exemple 1. On obtient ainsi l'oxalate de 3-méthane-sulfonamido-N-[3-(4-pipéridinométhylpyrid-2-yloxy)propyl]benzamide.

Par neutralisation du produit ainsi obtenu avec de l'hydroxyde de sodium, on obtient le 3-méthanesulfonamido-N-[3-(4-pipéridinométhylpyrid-2-yloxy)propyl]benzamide.

EXEMPLES 20 et 21

En opérant comme décrit dans l'exemple 1, par réaction de 0,03 mol de 3-(3-pipéridinométhylphénoxy)propylamine avec 0,03 mol de 3-acétamidobenzoate de 4-nitrophényle et, respectivement, de 3-éthanesulfonamidobenzoate de 4-nitrophényle, on obtient :

- le 3-acétamido-N-[3-(3-pipéridinométhylphénoxy)propyl]benzamide, SR 58070; F. 140-143°C (exemple 20); et
- le 3-éthanesulfonamido-N-[3-(3-pipéridinométhylphénoxy)propyl]-benzamide, sous forme d'oxalate, SR 58079A; F. 155-157°C (exemple 21).

EXEMPLES 22 à 39

En opérant comme décrit dans l'exemple 1, par réaction de 0,03 mol de 3-(3-butylaminométhylphénoxy)propylamine, de 3-(3-allylaminométhylphénoxy)propylamine, de 3-(3-propargylaminométhyl-phénoxy)propylamine, de 3-[(2-hydroxyéthyl)aminométhylphénoxy]-propylamine, de 3-(3-benzylaminométhylphénoxy)propylamine, de 3-(3-cyclopropylméthylaminométhylphénoxy)propylamine, de 3-(3-phényl-aminométhylphénoxy)propylamine, de 3-(3-cyclopentylaminométhylphénoxy)-propylamine, de 3-(3-diallylaminométhylphénoxy)propylamine, de 3-(3-dipropargylaminométhylphénoxy)propylamine, de 3-[3-di(2-hydroxyéthyl)-aminométhylphénoxy]propylamine, de 3-[3-di(2-méthoxyéthyl)aminométhyl-phénoxy]propylamine, de 3-[(méthylcyclopropylméthyl)aminométhyl-phénoxy]propylamine, de 3-[4-(2-hydroxyéthylpipérazino)méthylphénoxy]-propylamine, de 3-(3,4,5-triméthylpipérazinométhylphénoxy)propylamine, de 3-(3,4-diméthylpipérazinométhylphénoxy)propylamine, de 3-(3,5-diméthylpipéridinométhylphénoxy)propylamine et, respectivement, de 3-[4-(2-hydroxyéthyl)-3-méthylpipérazinométhylphénoxy]propylamine avec 0,03 mol de 3-méthanesulfonamidobenzoate de 4-nitrophényle, on obtient, respectivement :

- le 3-méthanesulfonamido-N-[3-(3-butylaminométhylphénoxy)propyl]-benzamide (exemple 22);
- le 3-méthanesulfonamido-N-[3-(3-allylaminométhylphénoxy)propyl]-benzamide (exemple 23);
- le 3-méthanesulfonamido-N-[3-(3-propargylaminométhylphénoxy)propyl]-benzamide (exemple 24);
- le 3-méthanesulfonamido-N-[3-[(2-hydroxyéthyl)aminométhylphénoxy]-propyl]benzamide (exemple 25);
- le 3-méthanesulfonamido-N-[3-(3-benzylaminométhylphénoxy)propyl]-benzamide (exemple 26);
- le 3-méthanesulfonamido-N-[3-(3-cyclopropylméthylaminométhyl-phénoxy)propyl]benzamide (exemple 27);

- le 3-méthanesulfonamido-N-[3-(3-phénylaminométhylphénoxy,propyl]-benzamide (exemple 28);
- le 3-méthanesulfonamido-N-[3-(3-cyclopentylaminométhylphénoxy)-propyl]benzamide (exemple 29);
- le 3-méthanesulfonamido-N-[3-(3-diallylaminométhylphénoxy)propyl]-benzamide (exemple 30);
- le 3-méthanesulfonamido-N-[3-(3-dipropargylaminométhylphénoxy)-propyl]benzamide (exemple 31);
- le 3-méthanesulfonamido-N-[3-[3-di(2-hydroxyéthyl)aminométhyl-phénoxy]propyl]benzamide (exemple 32);
- le 3-méthanesulfonamido-N-[3-[3-di(2-méthoxyéthyl)aminométhyl-phénoxy]propyl]benzamide (exemple 33);
- le 3-méthanesulfonamido-N-[3-[(méthyl-cyclopropylméthyl)amino-méthylphénoxy]propyl]benzamide (exemple 34);
- le 3-méthanesulfonamido-N-[3-[4-(2-hydroxyéthylpipérazino)méthyl-phénoxy]propyl]benzamide (exemple 35);
- le 3-méthanesulfonamido-N-[3-(3,4,5-triméthylpipérazinométhyl-phénoxy)propyl]benzamide (exemple 36);
- le 3-méthanesulfonamido-N-[3-(3,4-diméthylpipérazinométhylphénoxy)-propyl]benzamide (exemple 37);
- le 3-méthanesulfonamido-N-[3-(3,5-diméthylpipéridinométhylphénoxy)-propyl]benzamide (exemple 38); et, respectivement,
- le 3-méthanesulfonamido-N-[3-[4-(2-hydroxyéthyl)-3-méthylpipérazino-méthylphénoxy]propyl]benzamide (exemple 39).

EXEMPLE 40

Comprimés à base d'un des composés décrits dans les exemples 1 à 39, ayant la composition suivante :

| | |
|---|---|
| principe actif | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

On mélange intimement dans une mélangeuse-pétrisseuse tous les ingrédients, sauf le lubrifiant, pendant 15 min, puis on pétrit par addition graduelle d'eau. On fait passer la masse à travers un tamis de 1,25 mm. On sèche le granulé dans une étuve à ventilation

forcée jusqu'à ce qu'on obtienne une humidité résiduelle relativement réduite (environ 2%). On uniformise le granulé, on ajoute le lubrifiant et l'on forme des comprimés par compression. Poids d'un comprimé : 300 mg.

De la même façon, on prépare les comprimés contenant 250 mg de principe actif.

REVENDICATIONS

1 - Amides N-substitués de formule

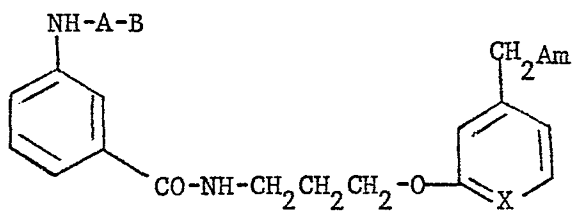

dans laquelle X représente un atome d'azote ou un groupe CH; Am représente un groupe amino substitué par un alkyle inférieur, par un alkyle inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant de 3 à 6 atomes de carbone; un groupe amino substitué par deux groupes alkényle inférieur ou alkynyle inférieur, identiques ou différents; un groupe amino substitué par deux groupes (hydroxy)-alkyle inférieur ou (alkoxy inférieur)alkyle inférieur, identiques ou différents; un groupe dialkylamino inférieur; un groupe dialkylamino inférieur substitué par un groupe phényle, cycloalkyle ayant 3 à 6 atomes de carbone, méthoxyle, ou hydroxyle; un groupe pyrrolidino; un groupe morpholino; un groupe thiomorpholino; un groupe hexaméthylèneimino; un groupe pipéridino; un groupe pipéridino substitué par un groupe méthyle, hydroxyle ou hydroxyméthyle ou par deux groupes méthyle; un groupe pipérazino substitué en position 4 par un groupe alkyle inférieur ou (hydroxy)alkyle inférieur; ou un groupe pipérazino substitué en position 4 par un groupe alkyle inférieur ou (hydroxy)alkyle inférieur et portant aussi un groupe méthyle dans les positions 3 et/ou 5; A est CO ou $SO_2$ et B est un groupe alkyle contenant 1 à 6 atomes de carbone, un groupe phényle, un groupe pyridyle ou un groupe pyridyle 1-oxyde, ainsi que leurs sels pharmaceutiquement acceptables.

2 - Le 3-benzènesulfonamido-N-[3-(3-pyrrolidino-méthylphénoxy)propyl]benzamide et ses sels pharmaceutiquement acceptables.

3 - Le 3-[(3-pyridine-1-oxyde)carboxamido]-N-[3-(3-diméthylaminométhylphénoxy)propyl]benzamide et ses sels pharmaceutiquement acceptables.

4 - Le 3-méthanesulfonamido-N-[3-(3-pipéridinométhylphénoxy)propyl]benzamide et ses sels pharmaceutiquement acceptables.

5 - Le 3-acétamido-N-[3-(3-pyrrolidinométhylphénoxy,-propyl]benzamide et ses sels pharmaceutiquement acceptables.

6 - Le 3-éthanesulfonamido-N-[3-(3-pipéridinométhylphénoxy)propyl]benzamide et ses sels pharmaceutiquement acceptables.

7 - Le 3-éthanesulfonamido-N-[3-[3-(3-méthylpipéridinométhyl)phénoxy]propyl]benzamide et ses sels pharmaceutiquement acceptables.

8 - Procédé pour la préparation d'amides N-substitués de formule

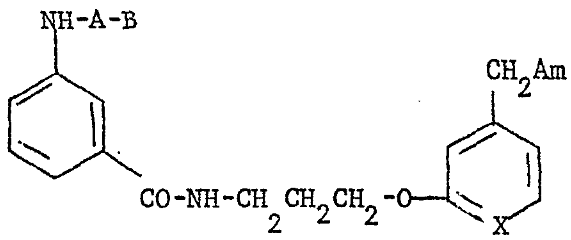

dans laquelle X représente un atome d'azote ou un groupe CH; Am représente un groupe amino substitué par un alkyle inférieur, par un alkyle inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant de 3 à 6 atomes de carbone; un groupe amino substitué par deux groupes alkényle inférieur ou alkynyle inférieur, identiques ou différents; un groupe amino substitué par deux groupes (hydroxy)alkyle inférieur ou (alkoxy inférieur)alkyle inférieur, identiques ou différents; un groupe dialkylamino inférieur; un groupe dialkylamino inférieur substitué par un groupe phényle, cycloalkyle ayant 3 à 6 atomes de carbone, méthoxyle, ou hydroxyle; un groupe pyrrolidino; un groupe morpholino; un groupe thiomorpholino; un groupe hexaméthylèneimino; un groupe pipéridino; un groupe pipéridino substitué par un groupe méthyle, hydroxyle ou hydroxyméthyle ou par deux groupes méthyle; un groupe pipérazino substitué en position 4 par un groupe alkyle inférieur ou (hydroxy)alkyle inférieur; ou un

groupe pipérazino substitué en position 4 par un groupe alkyle inférieur ou (hydroxy)alkyle inférieur et portant aussi un groupe méthyle dans les positions 3 et/ou 5; A est CO ou $SO_2$ et B est un groupe alkyle contenant 1 à 6 atomes de carbone, un groupe phényle, un groupe pyridyle ou un groupe pyridyle 1-oxyde et de leurs sels pharmaceutiquement acceptables, caractérisé en ce qu'on traite une amine de formule

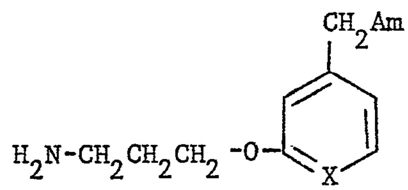

dans laquelle X et Am sont tels que définis ci-dessus, avec un dérivé fonctionnel de l'acide carboxylique de formule

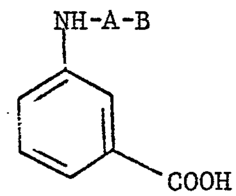

dans laquelle A et B sont tels que définis ci-dessus, dans un solvant organique, à une température comprise entre 0°C et la température d'ébullition du solvant employé et l'on transforme éventuellement le produit ainsi obtenu dans ses sels pharmaceutiquement acceptables.

9 - Procédé selon la revendication 8, caractérisé en ce que comme dérivé fonctionnel on utilise l'acide libre activé, l'anhydride, un anhydride mixte, le chlorure ou un ester actif.

10 - Procédé selon la revendication 8, caractérisé en ce que le dérivé fonctionnel a la formule

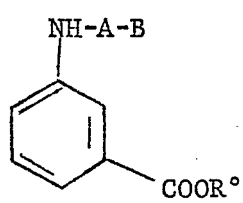

dans laquelle A et B sont tels que définis dans la revendication 8 et R° représente un groupe nitrophényle, méthoxyphényle, trityle, benzhydryle.

11 - Procédé selon une des revendications 8 à 10, caractérisé en ce qu'on isole le produit final sous forme d'un de ses sels et l'on libère la base libre dudit sel par neutralisation à l'aide d'un hydroxyde ou carbonate alcalin.

12 - Composition pharmaceutique renfermant, en tant que principe actif, un composé selon une des revendications 1 à 7.

13 - Composition pharmaceutique sous forme d'unité de dosage à action $H_2$-bloquante renfermant de 10 à 1000 mg d'un produit selon une des revendications 1 à 7 en mélange avec un excipient pharmaceutique.

14 - Composition selon la revendication 13, renfermant de 50 à 500 mg d'un produit selon une des revendications 1 à 7.

REVENDICATIONS

1 - Procédé pour la préparation de composés de formule :

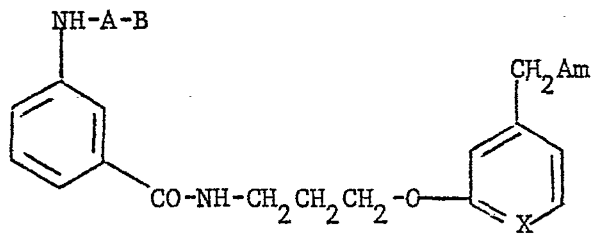

dans laquelle X représente un atome d'azote ou un groupe CH; Am représente un groupe amino substitué par un alkyle inférieur, par un alkyle inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant de 3 à 6 atomes de carbone; un groupe amino substitué par deux groupes alkényle inférieur ou alkynyle inférieur, identiques ou différents; un groupe amino substitué par deux groupes (hydroxy)alkyle inférieur ou (alkoxy inférieur)alkyle inférieur, identiques ou différents; un groupe dialkylamino inférieur; un groupe dialkylamino inférieur substitué par un groupe phényle, cycloalkyle ayant 3 à 6 atomes de carbone, méthoxyle ou hydroxyle; un groupe pyrrolidino; un groupe morpholino; un groupe thiomorpholine; on groupe hexaméthylèneimino; un groupe pipéridino; un groupe pipéridino substitué par un groupe méthyle, hydroxyle ou hydroxyméthyle ou par deux groupes méthyle; un groupe pipérazino substitué en position 4 par un groupe alkyle inférieur ou (hydroxy)alkyle inférieur; ou un groupe pipérazino substitué en position 4 par un groupe alkyle inférieur ou (hydroxy)alkyle inférieur et portant aussi un groupe méthyle dans les positions 3 et/ou 5; A est CO ou $SO_2$ et B est un groupe alkyle contenant 1 à 6 atomes de carbone, un groupe phényle, un groupe pyridyle ou un groupe pyridyle 1-oxyde, et de leurs sels pharmaceutiquement acceptables, caractérisé en ce qu'on traite une amine de formule :

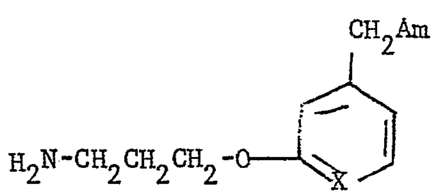

dans laquelle X et Am sont tels que définis ci-dessus, avec un dérivé fonctionnel de l'acide carboxylique de formule :

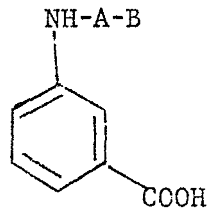

dans laquelle A et B sont tels que définis ci-dessus, dans un solvant organique, à une température comprise entre 0°C et la température d'ébullition du solvant employé et l'on transforme éventuellement le produit ainsi obtenu dans ses sels pharmaceutiquement acceptables.

2 - Procédé selon la revendication 1, caractérisé en ce que comme dérivé fonctionnel on utilise l'acide libre activé, l'anhydride, un anhydride mixte, le chlorure ou un ester actif.

3 - Procédé selon la revendication 1, caractérisé en ce que le dérivé fonctionnel a la formule :

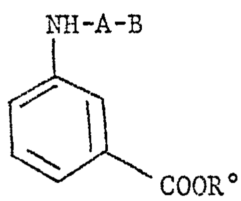

dans laquelle A et B sont tels que définis dans la revendication 1 et R° représente un groupe nitrophényle, méthoxyphényle, trityle, benzhydryle.

4 - Procédé selon une des revendications 1 à 3, caractérisé en ce qu'on isole le produit final sous forme d'un de ses sels et l'on libère la base libre dudit sel par neutralisation à l'aide d'un hydroxyde ou carbonate alcalin.